# EUROPÄISCHE PATENTSCHRIFT

(11) **EP 0 449 269 B1**
(45) Veröffentlichungstag und Bekanntmachung des Hinweises auf die Patenterteilung: **15.05.1996**
(21) Anmeldenummer: 91104912.0
(22) Anmeldetag: 27.03.1991
(51) Int. Cl.: G01N 33/68, G01N 33/566, G01N 33/543, G01N 33/547, G01N 33/58, G01N 33/574

(54) **Verfahren zum Nachweis von phosphoryliertes Tyrosin enthaltenden Proteinen**
Method for detection of phosphorylated tyrosine containing proteins
Méthode pour la détection des protéines contenant tyrosine phosphorylée

(30) Priorität: 27.03.1990 DE 4009848
(43) Veröffentlichungstag der Anmeldung: 02.10.1991
(73) Patentinhaber: BOEHRINGER MANNHEIM GMBH, D-68298 Mannheim (DE)
(72) Erfinder: Bodenmüller, Heinz, Dr., W-8132 Tutzing (DE); Dessauer, Andreas, Dr., W-8132 Tutzing (DE)
(74) Vertreter: Huber, Bernhard, Dipl.-Chem.

(56) Entgegenhaltungen:
- EP-A- 0 322 768
- EP-A- 0 322 813
- EP-A- 0 353 895
- EP-A- 0 356 964
- US-A- 4 543 439
- US-A- 4 758 522
- CHEMICAL ABSTRACTS, Band 112, Nr. 1, 1. Januar 1990, Columbus, OH (US); A.I. KHARITONENKOV et al., Seite 395, Nr. 3936b/
- NATURE, Band 294, Nr. 5842, 17. Dezember 1981, London (GB); A.H. ROSS et al./

## Beschreibung

Die Erfindung betrifft ein Verfahren zum Nachweis von phosphoryliertes Tyrosin enthaltenden Proteinen und eine dazu geeignete Reagenzien-Kombination.

Eine der wichtigsten Modifikationen von Proteinen ist ihre Phosphorylierung. Dabei werden Phosphorgruppen durch Einwirkung von Proteinkinasen an geeigneter Stelle auf das Protein übertragen. Häufig werden dabei die Hydroxylgruppen-haltigen Aminosäuren, und zwar überwiegend Serin oder Threonin phosphoryliert. Die Phosphorylierung von Serin und Threonin ist gegenüber der Phosphorylierung von Tyrosin in normalen Zellen bei weitem bevorzugt, wobei mehr als 99 % der Phosphatgruppen an Serin und Threonin vorliegen.

In transformierten Zellen wurde nun ein vermehrtes Auftreten von Phosphotyrosinkinase-Aktivität festgestellt. Dadurch kann sich der Anteil an Phosphotyrosin in transformierten Zellen um den Faktor 5 bis 70 erhöhen. Das Auftreten von Phosphortyrosin enthaltenden Proteinen wird daher mit der Transformation von Zellen in Verbindung gebracht. Es wird auch diskutiert, inwieweit der Phosphorylierungsgrad auf die Malignität einer Zelle hinweisen kann.

Weiterhin wurde beobachtet, daß viele Onkogenproteine eine Tyrosinkinase-Aktivität haben, d.h. sie können sich selbst und auch andere Proteine am Tyrosin phosphorylieren. Derartige Onkogenproteine treten sowohl plasmamembranständig als auch cytoplasmatisch auf. Da nun in der Regel Onkogene Faktoren darstellen, die die gleiche Funktion wie Protoonkogene in normalen Zellen haben, jedoch eine veränderte oder auch nicht mehr vorhandene Regulation haben, können die entsprechenden Produkte vermehrt in den Zellen auftreten. Es wurde nun bereits versucht, Produkte dieser Enzymaktivitäten nachzuweisen. Dazu wurden Versuche mit radioaktivem Phosphor ³P durchgeführt, die jedoch nur in Zellkulturen möglich sind und Untersuchungen am Tier oder gar Menschen unmöglich machen.

Es wurde auch bereits vorgeschlagen, phosphoryliertes Tyrosin enthaltende Proteine auf immunologischem Wege nachzuweisen. (z.b. in EP-A-0 322 813 und US-A-4 543 439). Die bisher bekannten Verfahren scheiterten jedoch daran, daß durch Kreuzreaktionen auch Proteine, die kein phosphoryliertes Tyrosin enthielten, erfaßt wurden.

Es war nun Aufgabe der Erfindung, ein Verfahren zur Verfügung zu stellen, mit dem an Tyrosin phosphorylierte Proteine in Körperflüssigkeiten in einfacher und schneller Weise nach Art und Menge bestimmt werden können.

Diese Aufgabe wird gelöst durch ein Verfahren zum Nachweis von phosphoryliertes Tyrosin enthaltenden Proteinen, das dadurch gekennzeichnet ist, daß man die Probe einer Körperflüssigkeit mit mindestens zwei Rezeptoren R₁ und R₂ inkubiert, wobei durch Bindung mindestens der Rezeptoren R₁ und R₂ mit der in der Probelösung nachzuweisenden Substanz eine Signaländerung erzeugt wird und wobei jeweils einer der beiden Rezeptoren einen mit dem nachzuweisenden Protein spezifisch bindefähigen Antikörper oder dessen Derivat enthält und der andere Rezeptor einen spezifisch mit phosphoryliertem Tyrosin bindefähigen Antikörper oder dessen Derivat enthält und die durch die Bindung verursachte Signaländerung in der Probe bestimmt.

Mit dem erfindungsgemäßen Verfahren ist es möglich, phosphoryliertes Tyrosin enthaltende Proteine genau und reproduzierbar in Körperflüssigkeiten nachzuweisen. Durch die Kombination zweier verschiedener Antikörper kann der Phosphorylierungsgrad und die Art des phosphorylierten Proteins schnell und einfach bestimmt werden.

Das Nachweisverfahren wird in Körperflüssigkeiten, bevorzugt Serum, durchgeführt. Für die Durchführung des erfindungsgemäß vorgesehenen immunologischen Bestimmungsverfahrens sind sehr viele Varianten bekannt, die alle hier geeignet sind. So können zwei oder drei oder auch mehr Rezeptoren verwendet werden und die Inkubation mit den einzelnen Rezeptoren kann in verschiedener Reihenfolge in homogener oder heterogener Phase erfolgen. Diese Verfahrensvarianten sind dem Fachmann bekannt und bedürfen hier keiner näheren Erläuterung.

Ausgewertet wird jeweils eine durch Bindung von mindestens zwei Rezeptoren mit der in der Probelösung nachzuweisenden Substanz erfolgende Signaländerung. Diese Signaländerung kann abhängig von dem verwendeten System z.B. eine Farbänderung, Trübungsänderung oder Fluoreszenzänderung sein. In jedem Fall werden mindestens zwei Rezeptoren eingesetzt, wobei die Signaländerung nur dann auftritt, wenn sowohl R₁ als auch R₂ an die nachzuweisende Substanz gebunden sind. Der Nachweis kann mit homogenen Verfahren wie z.B. dem Agglutinationsassay erfolgen, wobei als Rezeptoren beschichtete Partikel wie z.B. Latexpartikel oder Erythrozyten verwendet werden, die durch Bindung an die nachzuweisende Substanz vernetzen und dadurch agglutinieren oder mit heterogenen Verfahren wie beispielsweise einem Sandwich-Immunoassay.

In jedem Fall werden zwei Rezeptoren verwendet, von denen einer einen mit dem nachzuweisenden Protein spezifisch bindefähigen Antikörper oder dessen Derivat und der andere einen mit phosphoryliertem Tyrosin bindefähigen Antikörper oder dessen Derivat enthält.

Der für den Rezeptor R₁ verwendete Antikörper ist bevorzugt ein gegen das nachzuweisende Protein gerichteter Antikörper bzw. dessen Derivat. Als Proteine werden erfindungsgemäß solche nachgewiesen, die in Tumorgeweben häufig vorkommen und Substrate für Phosphotyrosinkinasen sind. Als Beispiele sind zu nennen:
Rezeptoren von Proteohormonen mit autokatalytischer Phosphorylierung wie Epideral derived growth factor - R., Platelet derived growth factor - R., Insulin - R., Insulin like growth factor - R., Nerve growth factor u.a.; Glykolytische Enzyme wie Isoenzyme der Enolase; Phosphoglyceratmutase; Laktatdehydrogenase (Nature 302, 218-223, 1983); Vesikelproteine wie Synaptophysin; Proteine mit Molekulargewicht 30 kD, 94 kD und 105 kD (PNAS 85, 762- 766, 1988); Phosphorylierte Onkogene wie v-fms (gp 140), pp60 c-src, HER-2/neu, Polyomavirus middle T Protein (56 kD) (Mol. Cell Biol. 8, 176-185, 1988) sowie weitere Onkogenproteine, die Abkömmlinge von Hormonrezeptoren sind; Zellskelettproteine wie Vinculin (Sci. Am. 246, 68-78, 1982) oder Calpactin (Mol. Cell. Biol. 6, 2745-2751, 1986); Proteine in Tumorzellinien, z.B. mit Molekulargewichten: 150 kD, 130 kD, 110 kD, 170 kD, 100 kD, 80 kD, 210 kD, 70 kD, 60 kD (Int. J. Cancer 39, 482-487, 1987). Bevorzugt werden für die Bestimmung solche Proteine ausgewählt, in denen das Phosphotyrosin stabil ist, da in Serum vorhandene Phosphatasen Phosphotyrosin in machen Proteinen dephosphorylieren können. Als stabile, gut nachzuweisende Proteine sind Synaptophysin, ein N-glykosyliertes Protein, das fest an die Vesikelmembran gebunden ist und üblicherweise in neurosekretorischen und neuroendokrinen Zellen vorkommt, Chromogranin A, ein saures, lösliches Protein, welches häufig in Granula sekretorischer Zellen vorkommt und Cytokeratine, Intermediär-Filament-Proteine, die Bestandteile des Cytoskeletts von Epitelzellen sind, besonders geeignet. Erfindungsgemäß werden daher die gegen diese Proteine gerichteten Antikörper bevorzugt verwendet.

Rezeptor R₁ ist abhängig vom verwenden Verfahren so derivatisiert, daß er entweder die Bindung an die feste Phase vermittelt, wenn es sich um einen heterogenen Immunoassay handelt oder in anderer Weise derivatisiert, wenn es sich um einen homogenen Immunoassay handelt, beispielsweise durch Bindung an Latexpartikel oder an Modulatorsubstanzen.

Bevorzugt erfolgt die erfindungsgemäße Bestimmung als Sandwich-Immunoassay. Dazu wird Rezeptor R₁ immobilisierbar gemacht und gleichzeitig oder sequenziell mit der Probelösung umgesetzt. Anschließend wird Rezeptor R₂ zugegeben. Es bilden sich Komplexe aus dem immobilisierten Rezeptor R₁, der nachzuweisenden Substanz sowie Rezeptor R₂. Nur Komplexe, die an die Festphase gebunden sind und eine Markierung tragen, gehen in die Auswertung ein.

In dieser Ausführungsform enthält der Rezeptor R₁ einen der beiden erfindungsgemäß verwendeten Antikörper bzw. dessen Derivat und vermittelt die Bindung an die feste Phase. Dazu kann Rezeptor R₁ entweder direkt oder über einen Spacer an eine feste Phase gebunden sein oder aber immobilisierbar sein. In einer bevorzugten Ausführungsform ist Rezeptor R₁ ein Konjugat aus einem Antikörper und einer spezifisch bindefähigen Substanz. Der mit der spezifisch bindefähigen Substanz bindefähige Partner ist an eine Festphase gebunden. Als spezifisch bindefähige Paare können beispielsweise Antigen-Antikörper; Hapten-Antikörper; Biotin-Antibiotin-Antikörper; Biotin-Avidin; Biotin-Streptavidin; Protein A-Immun-γ-Globulin genannt werden. Besonders bevorzugt wird in dieser Ausführungsform als R₁ ein Konjugat des Antikörpers mit Biotin und als Festphase eine Matrix verwendet, die an ihrer Oberfläche Streptavidin trägt. Durch Bindung des Biotins mit dem Streptavidin erfolgt dann die Immobilisierung des monoklonalen Antikörpers. Bevorzugt ist auch eine Ausführungsform, bei der an der Oberfläche der festen Phase Antikörper gegen den Fc-Teil der verwendeten monoklonalen Antikörper oder Protein-A-Moleküle gebunden werden, wobei dann durch Bindung der Fc-Teile der monoklonalen Antikörper die Immobilisierung erfolgt.

In einer weiteren bevorzugten Ausführungsform sind an eine Matrix Biotinmoleküle gebunden und als Rezeptor R₁ wird ein Konjugat aus Biotin und dem Antikörper verwendet. Die Immobilisierung kann dann nach Durchführung der immunologischen Reaktion durch Zugabe von Streptavidin erfolgen.

Als feste Phase sind die in immunologischen Verfahren üblicherweise verwendeten Materialien geeignet. Beispielsweise können Polymermaterialien oder auch cellulosehaltige Materialien sowie Glas verwendet werden. Als besonders geeignet haben sich Polystyrol, Polymethacrylat, Teflon™, Polyamid, Copolymere aus Styrol und Acrylnitril, Glas- und Celluloseprodukte erwiesen. Die Matrix kann in beliebiger Form vorliegen, z.B. als Röhrchen, Mikrotiterplatte, Kugel, Film, Pulver, Körnchen oder Faservlies. Geeignet sind beispielsweise nach einem in der Patentanmeldung EP-A 0 269 092 beschriebenen Verfahren erhaltene Festphasen.

Rezeptor R₂ enthält jeweils den anderen Antikörper, der erfindungsgemäß notwendig ist, oder dessen Derivat. Weiterhin ist Rezeptor R₂ so aufgebaut, daß er zu einer Signaländerung beitragen kann. Bei Verwendung für einen homogenen oder heterogenen Immunoassay unter Verwendung von Markierungen ist Rezeptor R₂ markiert. Als Markierung sind radioaktive Substanzen, Enzyme, fluoreszierende und chemilumineszierende Substanzen geeignet. Der Nachweis der Markierung erfolgt dabei nach bekannten Methoden. Bevorzugt wird als Markierung ein Enzym eingesetzt. Als Enzyme geeignet sind insbesondere Peroxidase, alkalische Phosphatase und β-Galactosidase. Der Nachweis des Enzyms erfolgt durch Zugabe eines Substrats und Messung der gebildeten Farbe. In anderen Varianten von Immunoassays ist Rezeptor R₂ gebunden an beschichtete Partikel.

Bevorzugt enthält Rezeptor R₂ als Antikörper einen spezifisch mit phosphoryliertem Tyrosin bindefähigen Antikörper. Ein spezifisch mit phosphoryliertem Tyrosin bindefähiger Antikörper kann erhalten werden, indem ein geeigneter Organismus mit Phosphotyramin in bekannter Weise immunisiert wird, Milzzellen gewonnen werden, die nach bekannten Verfahren mit Myelomzellen fusioniert werden und dann die mit Phosphotyrosin bindefähigen Klone selektioniert und gezüchtet werden. Zur Immunisierung wird Phosphotyramin an succinylierte Trägerproteine gebunden, wobei keyhole limpet hemocyanin (KLH) und Rinderserumalbumin bevorzugt als Trägerproteine eingesetzt werden. Besonders bevorzugt wird der von der Zellinie ECACC 90031904 produzierte monoklonale Antikörper 3-365-10 verwendet.

Bei Inkubation der Körperflüssigkeit mit den beiden Rezeptoren bilden sich Komplexe aus R₁, phosphoryliertes Tyrosin enthaltendem Protein und R₂. Nur Komplexe, in denen alle drei Komponenten gebunden sind, können eine Signaländerung bewirken. Auf diese Weise werden einerseits nur eine bestimmte Art von Proteinen und andererseits nur deren an Tyrosin gebundene Phosphorgruppen spezifisch erfaßt, da nur die entsprechenden Proteine mit den beiden spezifischen Antikörpern bindefähig sind. In der bevorzugten Ausführungsform des Sandwich-Immunoassays können nur Komplexe, an denen R₁ gebunden ist, immobilisiert werden und nur Komplexe, an denen weiterhin R₂ gebunden ist, über die Markierung ausgewertet werden.

Mit dem erfindungsgemäßen Verfahren kann das Vorhandensein von bestimmten Proteinen nachgewiesen werden, die zwei Epitope enthalten, die mit den beiden eingesetzten Antikörpern bindefähig sind. Derartige Fragmente entstehen überwiegend in Tumorgewebe.

Ein weiterer Gegenstand der Erfindung ist ein Reagenz zum Nachweis von phosphoryliertes Tyrosin enthaltenden Proteinen, das dadurch gekennzeichnet ist, daß es mindestens zwei Rezeptoren R₁ und R₂ enthält, wobei einer der beiden Rezeptoren einen mit dem zu bestimmenden Protein bindefähigen Antikörper oder dessen Derivat und der andere Rezeptor einen mit phosphoryliertem Tyrosin spezifisch bindefähigen Antikörper oder dessen Derivat enthält.

Gegenstand der Erfindung ist auch die bei der European Collection of Animal Cell Cultures, Port Down (GB) hinterlegte Zellkultur ECACC 90031904, die Antikörper produziert, die mit phoshoryliertem Tyrosin spezifisch bindefähig sind.

Die Erfindung wird durch das folgende Beispiel erläutert.

### Beispiel

### Testansatz mit Synaptophysin als Antigen

Es wurde phosphoryliertes Synaptophysin in Körperflüssigkeiten in einem Sandwich-Enzym-Immunoassay mit einem Synaptophysin-spezifischen Fängerantikörper und einem Phosphotyrosin-spezifischen Detektionsantikörper nachgewiesen. Es wurden Mikrotiterplatten, beschichtet mit Streptavidin analog EP-A 0 269 092, mit dem von der Zellinie ECACC 89112801 produzierten Synaptophysin-spezifischen MAK SY 38 (2µg/ml) als Biotinkonjugat (Herstellung über Reaktion des Antikörpers mit D-Biotin-ε-aminocapronsäure-N-hydroxy-succinimidester) in einem Volumen von 100 nl PBS (8 g/l NaCl, 0,2 g/l KCl, 1,44 g/l Na₂HPO₄x2H₂O, 0,2 g/l KH₂PO₄) inkl. 5 µl Probe pro Napf während 2 h bei Raumtemperatur beschichtet. Nach dreimaligem Waschen mit 0.05% Tween 20/PBS erfolgte die Inkubation mit dem Konjugat eines Anti-Phosphotyrosin-Antikörpers gekoppelt an Peroxidase (166U/L Inkubationskonzentration) in PBS.

Nach 1h Inkubation bei Raumtemperatur wurde erneut dreimal gewaschen mit 0.05% Tween™ 20/PBS. Nach anschließender Inkubation (60 Minuten) mit dem Enzymsubstrat ABTS (2,2'-Azino-di-[3-ethyl-benzthiazolin-sulfonsäure(6)]-diammoniumsalz, 9,1 mmol/l ABTS, 1,47 mmol/l Natriumperborat, 100 mmol/l Phosphat-Citrat-Puffer pH 5,0) bei Raumtemperatur wurde bei 405 nm die Extinktion als Maß für die Analytkonzentration gemessen.

Analog erfolgten die Testansätze mit dem Fänger-MAK Anti-Chromogranin (Herstellung analog Am. J. Surgical Pathology 8 (1984) 607-614) (2 µg/ml), einem Anti-Cytokeratin-MAK mit den Hinterlegungsnummern ECACC 89036302 (3 µg/ml), sowie mit einem Anti-Calpactin-MAK [Herstellung analog Biochemistry 27 (1988) 2069-2076].

**Tabelle**

| Serumbewertung | | | | |
|---|---|---|---|---|
| | SY 38 x PTyr | | CA x PTyr | |
| | positiv | untersucht | positiv | untersucht |
| Gesunde | 0 | 6 | 0 | 6 |
| benigne Erkrankungen | 0 | 10 | 0 | 10 |
| maligne Erkrankungen | 9 | 50 | 9 | 50 |

| | CK x PTyr | | Calp. x PTyr | |
|---|---|---|---|---|
| | positiv | untersucht | positiv | untersucht |
| Gesunde | 0 | 5 | 0 | 6 |
| benigne Erkrankungen | 0 | 13 | 0 | 10 |
| maligne Erkrankungen | 4 | 19 | 2 | 12 |

- PTyr =: Antiphosphotyrosin-MAK (ECACC 90031904)
- SY 38 =: Antisynaptophysin-MAK (ECACC 89112801)
- CA =: Antichromogranin-MAK
- CK =: Anticytokeratin-MAK (ECACC 89030302)
- Calp. =: Anticalpactin-MAK

## Patentansprüche

1. Verfahren zum Nachweis von phosphoryliertes Tyrosin enthaltenden Proteinen,
**dadurch gekennzeichnet,**
daß man die Probe einer Körperflüssigkeit mit mindestens zwei Rezeptoren R₁ und R₂ inkubiert, wobei durch Bindung mindestens der Rezeptoren R₁ und R₂ mit der in der Probelösung nachzuweisenden Substanz eine Signaländerung erzeugt wird und wobei jeweils einer der beiden Rezeptoren einen mit dem nachzuweisenden Protein spezifisch bindefähigen Antikörper oder dessen Derivat enthält und der andere Rezeptor einen spezifisch mit phosphoryliertem Tyrosin bindefähigen Antikörper oder dessen Derivat enthält und die durch die Bindung verursachte Signaländerung in der Probe bestimmt.

2. Verfahren nach Anspruch 1, **dadurch gekennzeichnet,** daß man als Rezeptor R₁ einen Rezeptor verwendet, der die Bindung an eine feste Phase vermittelt und als Rezeptor R₂ einen markierten Rezeptor verwendet und die Markierung nach Trennung der festen von der flüssigen Phase in einer der beiden Phasen bestimmt.

3. Verfahren nach Anspruch 1 oder 2, **dadurch gekennzeichnet,** daß phosphoryliertes Tyrosin in Synaptophysin, Chromogranin A, Calpactin oder Cytokeratinen nachgewiesen wird und daß Rezeptor R₁ einen gegen jeweils eines dieser Proteine gerichteten Antikörper enthält.

4. Verfahren nach einem der vorhergehenden Ansprüche, **dadurch gekennzeichnet,** daß man als Antikörper gegen phosphoryliertes Tyrosin einen monoklonalen Antikörper verwendet, der erhalten wurde durch Immunisierung mit Phosphotyramin.

5. Verfahren nach einem der vorhergehenden Ansprüche, **dadurch gekennzeichnet,** daß man als gegen phosphoryliertes Tyrosin gerichteten Antikörper einen von der Zellinie ECACC 90031904 produzierten Antikörper oder dessen Derivat verwendet.

6. Verfahren nach einem der vorhergehenden Ansprüche, **dadurch gekennzeichnet,** daß man als feste Phase eine Matrix verwendet, die mit Streptavidin beschichtet ist und als Rezeptor R₁ ein Konjugat eines mit dem nachzuweisenden Protein spezifisch bindefähigen Antikörpers oder dessen Derivat und Biotin, wobei die Bindung des monoklonalen Antikörpers an die Festphase durch die Bindung von Biotin an Streptavidin erfolgt.

7. Verfahren nach einem der vorhergehenden Ansprüche, **dadurch gekennzeichnet,** daß man als Rezeptor R₂ einen mit phosphoryliertem Tyrosin spezifisch bindefähigen Antikörper oder dessen Derivat verwendet, der radioaktiv, mit einer fluoreszierenden oder chemilumineszierenden Substanz oder einem Enzym markiert ist.

8. Reagenzien-Kombination zum Nachweis von phosphoryliertes Tyrosin enthaltenden Proteinen, **dadurch gekennzeichnet,** daß sie mindestens zwei Rezeptoren R₁ und R₂ enthält, wobei einer der beiden Rezeptoren einen mit dem zu bestimmenden Protein spezifisch bindefähigen Antikörper oder dessen Derivat und der andere Rezeptor einen mit phosphoryliertem Tyrosin spezifisch bindefähigen Antikörper oder dessen Derivat enthält.

## Claims

1. Method for the detection of proteins containing phosphorylated tyrosine,
**wherein**
the sample of a body fluid is incubated with at least two receptors R₁ and R₂ in which a change in signal is produced by binding of at least the receptors R₁ and R₂ to the substance to be detected in the sample solution and in which in each case one of the two receptors contains an antibody or a derivative thereof capable of specifically binding to the protein to be detected and the other receptor contains an antibody or a derivative thereof capable of specifically binding to phosphorylated tyrosine and the signal change caused by the binding is determined in the sample.

2. Method as claimed in claim 1,
**wherein**
a receptor which mediates binding to a solid phase is used as the receptor R₁ and a labelled receptor is used as receptor R₂ and the label is determined in one of the two phases after separation of the solid from the liquid phase.

3. Method as claimed in claim 1 or 2,
**wherein**
phosphorylated tyrosine in synaptophysin, chromogranin A, calpactin or cytokeratins is detected and wherein receptor R₁ contains an antibody which in each case is directed towards one of these proteins.

4. Method as claimed in one of the previous claims,
**wherein**
a monoclonal antibody is used as the antibody against phosphorylated tyrosine which was obtained by immunization with phosphotyramine.

5. Method as claimed in one of the previous claims,
**wherein**
an antibody produced by the cell line ECACC 90031904 or a derivative thereof is used as the antibody directed against phosphorylated tyrosine.

6. Method as claimed in one of the previous claims,
**wherein**
a matrix is used as the solid phase which is coated with streptavidin and a conjugate of an antibody or a derivative thereof capable of specifically binding to the protein to be detected and biotin is used as the receptor R₁ in which the binding of the monoclonal antibody to the solid phase takes place by means of the binding of biotin to streptavidin.

7. Method as claimed in one of the previous claims,
**wherein**
an antibody or a derivative thereof capable of specifically binding to phosphorylated tyrosine is used as receptor R₂ which is labelled radioactively or with a fluorescent or chemiluminescent substance or with an enzyme.

8. Combination of reagents for the detection of proteins containing phosphorylated tyrosine,
**wherein**
it contains at least two receptors R₁ and R₂ in which one of the two receptors contains an antibody or a derivative thereof capable of specifically binding to the protein to be determined and the other receptor contains an antibody or a derivative thereof capable of specifically binding to phosphorylated tyrosine.

## Revendications

1. Procédé de détection de protéines contenant une tyrosine phosphorylée, caractérisé en ce que l'on met l'échantillon d'un liquide corporel à incuber avec au moins deux récepteurs R₁ et R₂, dans des conditions dans lesquelles la liaison d'au moins les récepteurs R₁ et R₂ avec la substance à détecter dans la solution d'échantillon entraîne la modification d'un signal, l'un des deux récepteurs contenant un anticorps ou un dérivé d'anticorps qui se lie de façon spécifique à la protéine à détecter et l'autre récepteur contenant un anticorps ou un dérivé d'anticorps qui se lie de façon spécifique à la tyrosine phosphorylée, et on détermine dans l'échantillon la modification du signal entraînée par la liaison.

2. Procédé selon la revendication 1, caractérisé en ce que l'on utilise comme récepteur R₁ un récepteur qui permet la fixation sur une phase solide et comme récepteur R₂ un récepteur marqué, et on dose le marqueur dans l'une des deux phases après avoir séparé la phase solide de la phase liquide.

3. Procédé selon la revendication 1 ou 2, caractérisé en ce que l'on détecte la tyrosine phosphorylée dans la synaptophysine, la chromogranine A, la calpactine ou des cytokératines, et le récepteur R₁ contient un anticorps dirigé de façon correspondante contre l'une des ces protéines.

4. Procédé selon l'une des revendications précédentes, caractérisé en ce que l'on utilise comme anticorps contre la tyrosine phosphorylée un anticorps monoclonal obtenu par immunisation avec de la phosphotyramine.

5. Procédé selon l'une des revendications précédentes, caractérisé en ce que l'on utilise comme anticorps dirigé contre la tyrosine phosphorylée un anticorps produit par la lignée cellulaire ECACC 90031904 ou l'un de ses dérivés.

6. Procédé selon l'une des revendications précédentes, caractérisé en ce que l'on utilise comme phase solide une matrice revêtue de streptavidine et comme récepteur R₁ un conjugué de biotine et d'un anticorps ou d'un dérivé d'anticorps se liant de façon spécifique avec la protéine à détecter, la fixation de l'anticorps monoclonal sur la phase solide s'effectuant par l'intermédiaire de la liaison de la biotine à la streptavidine.

7. Procédé selon l'une des revendications précédentes, caractérisé en ce que l'on utilise comme récepteur R₂ un anticorps ou un dérivé d'anticorps qui se lie de façon spécifique à la tyrosine phosphorylée et qui est marqué par une substance radioactive, fluorescente ou chimiluminescente ou par une enzyme.

8. Combinaison de réactifs pour la détection de protéines contenant une tyrosine phosphorylée, caractérisée en ce qu'elle comprend au moins deux récepteurs R₁ et R₂, l'un des deux récepteurs contenant un anticorps ou un dérivé d'anticorps qui se lie de façon spécifique à la protéine à détecter et l'autre récepteur contenant un anticorps ou un dérivé d'anticorps qui se lie de façon spécifique à la tyrosine phosphorylée.
